(19) Europäisches Patentamt
European Patent Office
Office européen des brevets

(11) Publication number: 0 080 814 B1

(12) # EUROPEAN PATENT SPECIFICATION

(45) Date of publication of patent specification: 15.10.86

(21) Application number: 82305948.0

(22) Date of filing: 09.11.82

(51) Int. Cl.$^4$: C 07 D 213/50, C 07 D 401/06, C 07 D 471/04, B 41 M 5/16, B 41 M 5/18 // (C07D471/04, 221:00, 221:00),(C07D471/04, 221:00, 209:00)

(54) ## Chromogenic compounds.

(30) Priority: 10.11.81 JP 178992/81

(43) Date of publication of application: 08.06.83 Bulletin 83/23

(45) Publication of the grant of the patent: 15.10.86 Bulletin 86/42

(84) Designated Contracting States: AT BE CH DE FR GB IT LI LU NL SE

(56) References cited:
DE-A-2 355 742
GB-A-1 211 393
GB-A-1 224 590

(73) Proprietor: YAMAMOTO KAGAKU GOSEI CO., LTD.
1 -43, Yuge-cho minami
Yao - Shi Ohsaka - Pref. (JP)

(72) Inventor: Kumagae, Yojiro
3-26, 1-chome Nishi-Mikuni
Yodogawa-ku Osaka-shi (JP)
Inventor: Iwasaki, Yasuhisa
110, 2-chome Minatsugaoka-Nishi
Sango-cho Ikoma-gun Nara-prefecture (JP)

(74) Representative: Roberts, Jonathan Winstanley et al
The Wiggins Teape Group Limited Group
Patents Dept. Butler's Court
Beaconsfield Buckinghamshire HP9 1RT (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to chromogenic compounds which can give intensely coloured reaction products with electron accepting (acidic) co-reactants. In particular it relates to diketopyridine compounds which form yellow, orange or similar colours with acidic co-reactants.

It has been long recognised that the availability of yellow or orange colour formers having coloured forms of high intensity and light fastness would be very useful in formulating chromogenic compositions for carbonless paper. An especial use is in pressure sensitive copying paper where a black image is desired as the "colour" black is usually generated by using a plurality of colour formers such as red, blue, yellow/orange and green/black. Currently available yellow colour formers are generally not as satisfactory as available red and blue colour formers, particularly by fading or changing hue under exposure to light. Thus, UK Patent Specification No. 1211393 describes *inter alia* 2-*N*-cyclohexylamino-7-chlorofluoran which has been widely used as a yellow-orange colour former in carbonless paper although it does not have especially good fade performance. UK Patent Specification No. 1416778 relates to bis(indol-3-yl)pyridine carboxylic acid lactones which are described as developing blue, purple or reddish purple colours with acidic co-reactants. Aryl(indol-3-yl)-substituted pyridyl ketones are referred to as intermediates. Additionally, UK Patent Specification No. 1224590 describes arylene, for example pyridylene, bis(phenoxy-substituted phenyl) ketones useful as lubricants or heat transfer fluids.

The present invention is based on the discovery that certain diketopyridines are capable of producing intense yellow or orange colours which show high light stability.

Accordingly, the present invention provides compounds having the general formula (I):

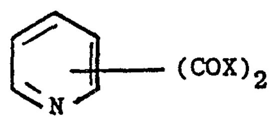

I

wherein X is:

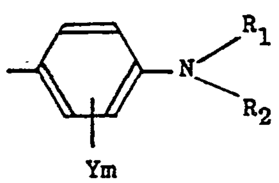
,
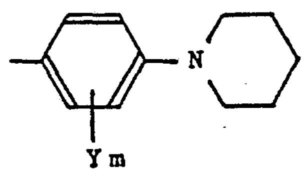
,
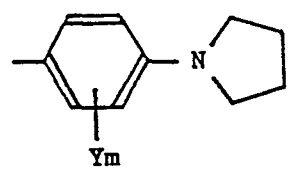
,

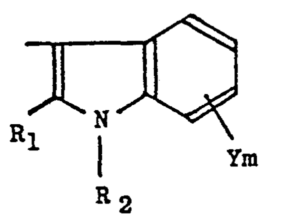
,
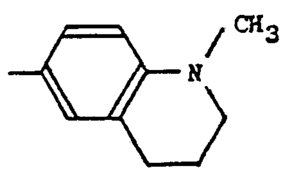
,
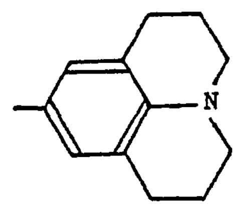
,

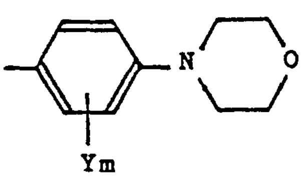
,
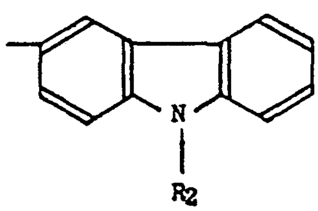
or
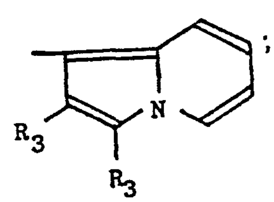
;

where Y is $C_1$ to $C_6$ alkyl, substituted $C_1$ to $C_6$ alkyl, $C_1$ to $C_6$ alkoxy, $C_1$ to $C_6$ alkyl dialkylamino, phenyl substituted phenyl, benzyl, substituted benzyl, halogen, a fused benzene ring or a substituted fused benzene ring;

m is O or an integer from 1 to 4; each $R_1$, $R_2$ and $R_3$ is independently $C_1$ to $C_{10}$ alkyl, substituted $C_1$ to $C_{10}$ alkyl, phenyl, substituted phenyl, benzyl, substituted benzyl or $C_5$ or $C_6$ cycloalkyl; and

wherein the substituent radicals in the "substituted" alkyl, alkoxy, phenyl, fused benzene ring or benzyl radical(s) is (are) $C_1$ to $C_{10}$ alkyl, halogen substituted $C_1$ to $C_4$ alkyl, $C_1$ to $C_4$ alkoxy $C_1$ to $C_4$ alkyl dialkylamino, halogen or cyano.

Preferably, independently each $R_2$ is independently a $C_1$ to $C_{10}$ alkyl, benzyl or cycloalkyl; each $R_3$ is independently $C_1$ to $C_4$ alkyl or phenyl; and where X includes two $R_1$ groups they are not both cycloalkyl.

The $C_1$ to $C_6$ alkyl radicals in the above-defined alkyl, alkoxy and dialkylamino groups may be the same or difference and are saturated, straight or branched chain, preferably $C_1$ to $C_4$, alkyl radicals. Examples of

such alkyl groups include methyl, ethyl, *n*-propyl, isopropyl and butyl; examples of such alkoxy groups include methoxy, ethoxy and isopropoxy and examples of such dialkylamino groups are dimethylamino, diethylamino, diisopropylamine and dibutylamino. The $C_1$ to $C_{10}$ alkyl groups are saturated, straight or branched chain, preferably $C_1$ to $C_8$, alkyl groups. Examples of such groups are methyl, ethyl, butyl and *n*-octyl groups.

Halogen atoms as or in the substituents can be fluorine, bromine, iodine or preferably chlorine.

Compounds of the formula (I) can be synthesized by reacting a pyridine carboxylic acid halide of the formula (II):

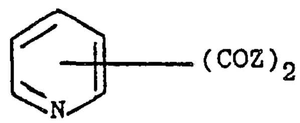

II

with a compound of the formula (III):

X—H (III)

wherein Z is chlorine or bromine and X is as defined above, in an organic solvent such as chloroform, carbon disulfide, dichloroethane, chlorobenzene or dichlorobenzene, in the presence of a Freidel-Crafts catalyst such as aluminium chloride or ferric chloride.

Compounds of the formula (I) in which the (COX) groups are located at adjacent positions on the pyridine nucleus ("ortho compounds") can also be synthesized by treating a leuco compound of one of the formulae (IV) to (VII):

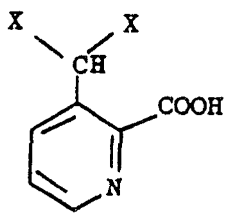
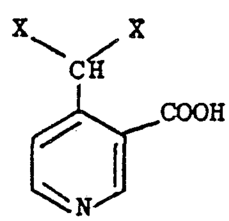
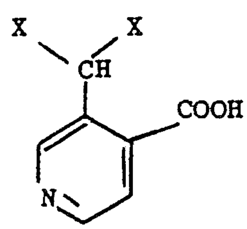
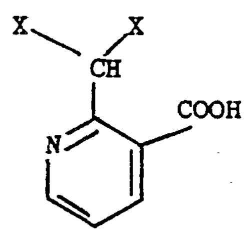

IV V VI VII

wherein X is:

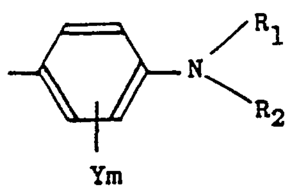 , 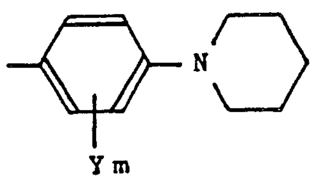 , 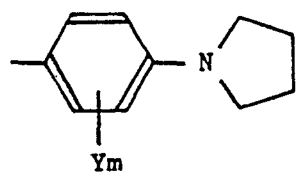 ,

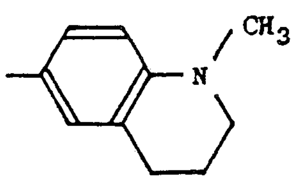 , 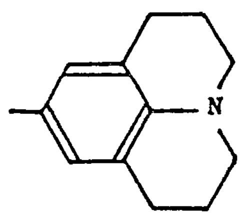 ,

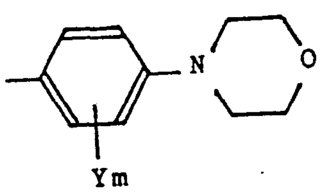 or 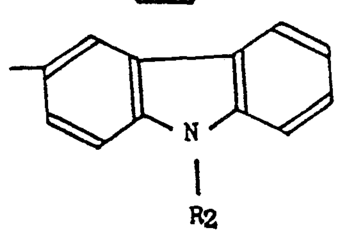

where Y, m, $R_1$ and $R_2$ are as defined above; with an acid anhydride such as acetic anhydride followed by an oxidizing agent such as ferric chloride or a chromate or dichromate. The leuco compounds (IV), (V), (VI) and (VII) can be synthesized by reacting the corresponding pyridine dicarboxylic acid anhydride (quinolinic acid anhydride for Compounds (IV) and (VII) and chinchomeroic acid anhydride for Compounds (IV) and (VII) with a compound of the formula XH, where X is as defined above, in the presence of an acid

anhydride to give an intermediate lactone and reducing the lactone, or the corresponding hydroxycarboxylic acid, e.g. with zinc and acid. As the result of XH with the pyridine carboxylic acid anhydride can take place at the carbon atom of either carboxyl, the result will typically be a mixture of isomers of the lactone and the leuco compound.

The subsequent reaction involves an overall intramolecular re-arrangement so that each pair of isomers gives a single corresponding product.

The chromogenic diketopyridine compounds of this invention are colourless or lightly coloured solids, which are stable to ambient atmospheric exposure. When these compounds are brought into reactive contact with electron accepting (acidic) materials they rapidly produce characteristically yellow, orange or reddish-orange coloured products which are very stable. Thus, these diketopyridine compounds are useful as colour formers in pressure sensitive copying systems, thermally responsive record material electrothermal recording paper and thermal ink.

Pressure sensitive copying systems provide a mark-forming system disposed on and/or within a sheet support material in which a colour former, a co-reactant and a liquid solvent in which either or both the colour former and co-reactants are soluble. The liquid solvent is present in a form in which it is isolated from either or both the colour former and co-reactant by a barrier which is pressure rupturable to permit local reactive contact between the solvent, colour former and co-reactant to form a coloured mark by reaction of the colour former and co-reactant. The pressure rupturable barrier is most commonly formed by microcapsules containing the liquid solvent. Typically the encapsuled solvent includes either the colour former or co-reactant in solution and, most usually, the solution is of the colour former. In the present invention the microencapsulation technique used in preparing pressure sensitive copying systems can be those known in the art, particularly those in current use. Such systems can be based on coacervation of natural or synthetic colloids or *in situ* polymerisation of monomers, whether or not by coacervation. Among the microencapsulation techniques which can be used in this invention are those described in US Patent Specifications Nos. 2800457, 3041289, 3533958, 3755190, 4001140 and 4100103 and UK Patent Specifications Nos. 1507739 and 2073132, to which reference can be made for process information.

Suitable solvents for preparing colour former containing microcapsules are those used in the art and include aromatic hydrocarbon solvents such as alkylated benzenes, naphthalenes and biphenyls; benzylated benzenes; and partially hydrogenated terphenyls, ester solvents such as phthalate and benzoate esters, long chain alcohols, phosphate ester solvents, and long chain aliphatic hydrocarbons such as kerosenes $C_9$ to $C_{14}$ alkanes) which are not themselves especially good solvents but are useful as diluents with other solvents.

The electron accepting or acidic co-reactant is usually either an inorganic acid reacting material or synthetic mineral especially activated clays e.g. attapulgite, bentonite and montmorillonite, acid washed reactive clays such as Silton clay (the word "Silton" is a Registered Trade Mark) as disclosed in US Specifications 3622364 and 3753761 or an organic acid reacting material including phenols and diphenols as are disclosed in US Specification 3539375; aromatic carboxyl acids such as salicylic acid and derivatives thereof, metal salts of aromatic carboxylic acids, especially zinc salts such as are disclosed in US Specification 4022936 and acidic organic polymers such as phenol-formaldehyde polymers such as disclosed in US Specification 3672935 and oil-soluble metal, especially zinc, salts of phenol-formaldehyde polymers as disclosed in US Specification 3732120.

The compounds of this invention are particularly useful in pressure-sensitive copying systems in which several colour formers are used together to give a substantially black image on reactive contact with an acidic coreactant by the combination of the characteristic colours of the individual colour formers. This general type of combination is as disclosed in US Specification 3525630.

Thus, in a further aspect the present invention provides a record material incorporating, as a colour former at least one chromogenic diketopyridine compound of the invention. The invention includes a manifold set of record material which comprises an upper sheet carrying on the lower surface thereof a coating of a microencapsulated solution of a chromogenic composition including at least one compound of this invention, a lower sheet carrying on the upper surface thereof a coating comprising an electron accepting (acidic) co-reactant and, optionally, one or more intermediate sheets each of which has on its upper surface a coating comprising an electron accepting (acidic) co-reactant and on its lower surface a coating comprising a microencapsulated solution of a chromogenic composition, including at least one compound of this invention. As has been indicated previously it is a particular feature of the invention that the chromogenic composition used in the record material of the invention includes a plurality of colour formers to give, on reaction with the co-reactant, a perceived black image.

The compounds of this invention can also be used in thermal record systems. Generally, in thermal record systems the colour former and co-reactant do not react at ambient temperature because both are solid. The colour forming reaction takes place when the record fluid, usually liquid. A binder is included to ensure adhesion to the substrate and uniform distribution of the solid reagents. Commonly other components are included e.g. fusible waxes to modify the fusion temperature, pigments either to colour or opacify the paper or to ensure rapid absorption of the coloured products and to reduce abrasion of and 'picking' of the paper by the thermal imaging element.

In a further embodiment the invention includes a thermally responsive record material which comprises a sheet substrate, typically of paper, having at least one surface thereof a coating comprising a

finely divided solid chromogenic composition including at least one compound of the invention and a finely divided solid electron accepting co-reactant uniformly distributed in a thermographically acceptable binder. Optionally the coating additionally comprises a finely divided, solid thermofusible wax to modify the fusion temperature of one or both of the colour former and co-reactant and may include surfactants and pigments.

As for pressure sensitive copying systems the chromogenic compositions can include a plurality of colour formmers. Preferably, such combinations produce a perceived black image when developed. Examples of such systems are disclosed in US Specifications 3539375 and 4181771.

Thermally responsive record material of this type is typically made by separately finely grinding the chromogenic composition, co-reactant and thermofusible wax, typically each together with a portion of an aqueous dispersion of the polymeric binder, combining the separate dispersions, optionally with the addition of other components in solution dispersion or other finely divided form, to form a coating composition, coating the sheet substrate and drying the composition. Typically the coated dried substrate is calendered to make the coated surface smooth to aid movement of the thermal imaging device over the surface.

The following Examples illustrate the invention. All parts and percentages are by weight unless otherwise stated.

Example 1

2,6-Bis(N-methyl, N-cyclohexyl-4'-aminobenzoyl)pyridine

4 g (0.02 mol of pyridine-2,6-carboxylic acid chloride and 5.7 g (0.043 mol) of aluminium chloride were dissolved with stirring at ambient temperature in 100 ml of dichloroene. The mixture was cooled in a water bath whilst 8 g (0.42 mol) of N-methyl-N-cycloahexylaniline were added dropwise. The reaction mixture was stirred ambient temperature for a further 20 hours and then quenched into 200 ml water, made alkaline, and the solvent and unreacted organic starting materials stripped off by steam distallation. The residue was extracted with 200 ml toluene, washed with hot water, filtered and evaporated to give 3.5 g (0.0072 mol) of 2,6-bis(N-methyl-N-cyclohexyl-4'-aminobenzoyl) pyridine of the formula VIII:

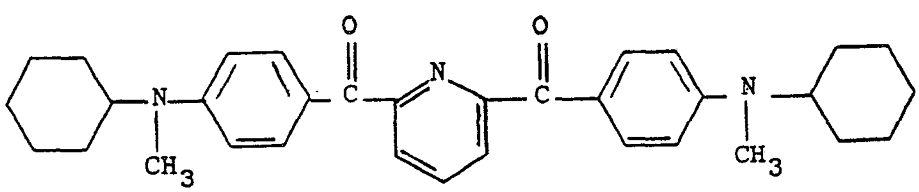

This compound is a pale yellow solid of melting point 168.5 to 170.5°C. An organic solvent solution of the product produces a yellow colour on Silton clay and silicia gel and an orange colour with phenolic resins and metal modified resins specifically the zinc salt of a phenol formaldehyde novolak resin made as described in US Patent 3732120.

Elemental analysis of the diketropyridine product gave the following result:

| | | C | H | N |
|---|---|---|---|---|
| Formula: $C_{33}H_{39}N_3O_2$ | Calculated | 77.75 | 7.73 | 8.25 |
| | Found | 77.67 | 7.65 | 8.13 |

Examples 2 to 17

Sixteen further 2,6-Bis-ketopyridine compounds of the formula (VIII):

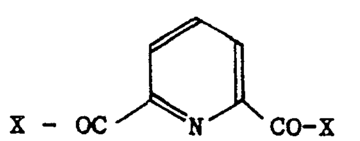

(VIII)

were prepared by a method similar to that of Example 1 but substituting appropriate starting compounds for the N-cyclohexyl-N-methylaniline used in Example 1. Table 1 below indicates the substituent "X", the melting point of the product and the colour obtained on Silton clay and with the zinc salt of a phenol formaldehyde novolak resin, referred to as "resin salt", for each of these Examples.

TABLE 1

| Ex. No. | X | M. Pt. (°C) | Colour on: | |
|---|---|---|---|---|
| | | | Silton® Clay 1) | Resin salt |
| 2 | 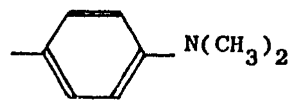 | 220.0–221.5 | Yellow | Orange |
| 3 | 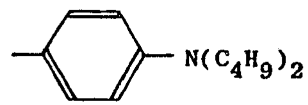 | 139.5–140.5 | Yellow | Orange |
| 4. | 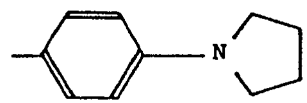 | 257.0–258.5 | Yellow | Orange |
| 5 | 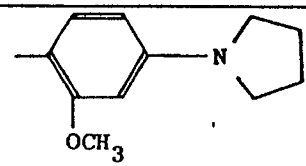 | 218.0–219.5 | Yellow | Orange |
| 6 | 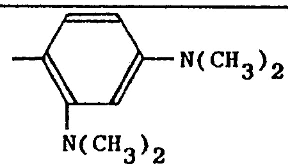 | 191.0–192.5 | Yellow | Reddish-Orange |

1) ® trademark.

TABLE 1 (Continued)

| Ex. No. | X | M. Pt. (°C) | Colour on: Silton Clay | Colour on: Resin salt |
|---|---|---|---|---|
| 7 | 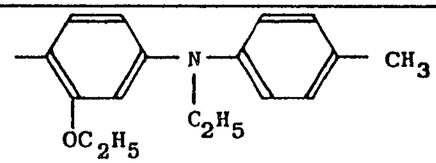 | 208.5–209.5 | Yellow | Orange |
| 8 | 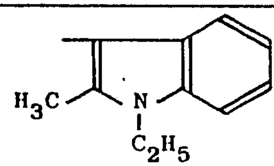 | 231.0–232.5 | Yellow | Yellow |
| 9 | 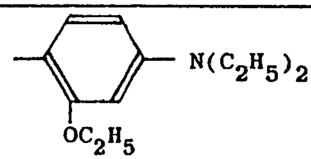 | 163.0–164.5 | Yellow | Yellowish-Orange |
| 10 | 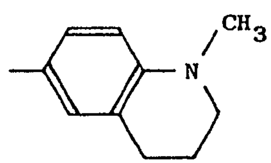 | 242.5–244.0 | Yellow | Orange |
| 11 | 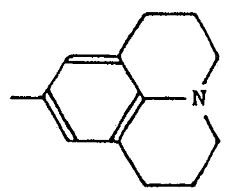 | 241.5–243.0 | Yellowish-Brown | Red |

TABLE 1 (Continued)

| Ex. No. | X | M. Pt. (°C) | Colour on: | |
|---|---|---|---|---|
| | | | Silton Clay | Resin salt |
| 12 | 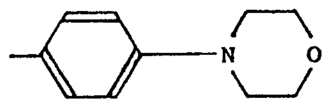 | 231.5–232.5 | Yellow | Brownish-Yellow |
| 13 | 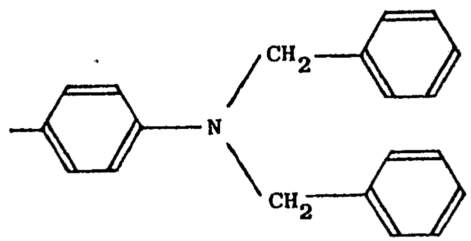 | 177.4–179.5 | Yellow | Brownish-Yellow |
| 14 | 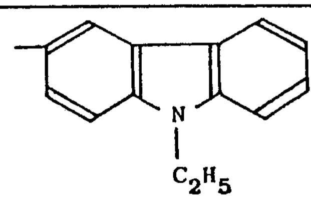 | 214.5–215.5 | Yellow | Yellow |
| 15 | 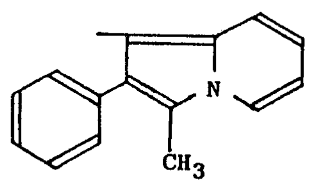 | 133.0–134.5 | Yellow | Orange |

TABLE 1 (Continued)

| Ex. No. | X | M. Pt. (°C) | Colour on: | |
|---|---|---|---|---|
| | | | Silton Clay | Resin salt |
| 16 | 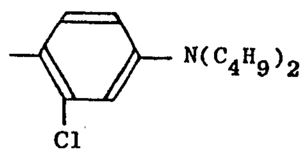 | 132.5–133.5 | Yellow | Orange |
| 17 | 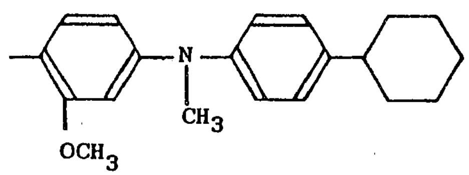 | 202.5–205.5 | Yellow | Orange |

Example 18

2,3-Bis(N,N-dimethyl-4'-aminobenzoyl)pyridine

35 g (0.29 mol) of N,N-dimethylaniline and 20 g (0.13 mol) of quinolinic acid anhydride were reacted in 50 ml of acetic acid anhydride were reacted in 50 ml of acetic anhydride at 80 to 90°C for about 4.5 hours. After completion of the reaction, the reaction mixture was made alkaline and then extracted with 200 ml of toluene at 85°C. The toluene solution was concentrated and the solid which separated out was filtered and dried to give 23 g of a mixture and the pair of isomers of an intermediate lactone compound (IX), 7,7-bis(N,N-dimethyl-4'-aminophenyl)-5,7-dihydrofuro[3,4-b]pyridine-5-one being the major isomer.

To a stirred mixture of 20 g of this intermediate product 70 ml of acetic acid and 6 g zinc powder under reflux were added dropwise 25 ml of 35% hydrochloric acid over a period of 2 hours. The mixture was refluxed for a further 1 hour, the reaction mixture was made alkaline and the insoluble material filtered off. The filtrate was neutralized and the solid precipitate was filtered, washed and dried to give 9.7 g (0.026 mol) of leuco intermediate Compound (X), the major isomer being bis-(N,N-dimethyl-4'-aminophenyl) (3-carboxypyridin-2-yl)methane.

A mixture of 5 g (0.013 mol) of Compound (X) and 7.5 g of acetic anhydride were stirred at 110°C for 9 hours under a nitrogen atmosphere. The reaction mixture was quenched into ice water, made alkaline and extracted with toleune at 80 to 85°C. The toleune solution was washed with hot water, filtered and evaporated to give 0.5 g of a deposit. This deposit was added to a solution of 1 ml of 35% hydrochloric cid and 0.5 g ferric chloride in 10 ml water and the mixture was stirred at 70°C for 1 hour.

This mixture was then made alkaline with soda ash ($Na_2CO_3$) and extracted with toluene. The toluene extract was washed with hot water, filtered and evaporated to give 0.2 g (0.00054 mol) of 2,3-bis(N,N-dimethyl-4'-amino-benzoyl)pyridine, Compound (XI). This product was pale yellow and melted with decomposition at 192.0 to 194.0°C. an organic solvent solution of this product have a yellow colour on silton clay and on a zinc salt of a phenol formaldehyde novolak resin.

Elemental analysis of Compound (XI) gave the following results:

| | | C | H | N |
|---|---|---|---|---|
| Formula: $C_{23}H_{23}N_5O_2$ | Calculated | 73.96 | 6.22 | 11.35 |
| | Found | 73.97 | 6.28 | 11.13 |

Example 19

Pressure sensitive copying paper

0.5 g of 2,6-bis (N-methyl-N-cyclohexyl-4'-aminobenzoyl)pyridine produced in Example 1 was dissolved in 12 ml of isopropylnapthalene (sold under the trade name KMCR by Kurcha Kagaku). This solution was microencapsulated by complex coacervation, using gum arabic and gelatin colloids, substantially by the method described in US Patent No. 2800457. The suspension of microcapsules was coated onto one side of a sheet of paper (the CB sheet) and dried. Other sheets of paper (CF sheets) were coated with the zinc salt of a phenol-formaldehyde novolak resin or with silton clay. Pairs comprising a CB sheet and a CF sheet were made up with the coatings facing and the CB sheet uppermost. Writing or typing on the upper (CB) sheet produced a yellow coloured copy image on the silton CF and an orange coloured copy image on the zinc novolak salt CF. Both images had good light fastness properties. Similar results were obtained using other solvents and other microencapsulation techniques.

Example 20

Thermally sensitive paper

A mixture of 3.5 g of 2,6-bis(N-methyl-N-Cyclohexyl-4'-aminobenzoyl) pyridine produced in Example 1, 15 g of a 10% aqueous solution of a polyvinyl alcohol and 6.5 g of water was ground in a ball mill for 24 hours to give Dispersion A. A mixture of 35 g of 4,4'isopropylidene-diphenol (Bisphenol A), 150 g of a 10% aqueous solution of polyvinyl alcohol and 65 g water was ground in a ball mill for 24 hours to give Dispersion B. A mixture of 3 parts of Dispersion A and 67 parts of Dispersion B was coated onto paper and dried. When the coated paper was heated with a hot stylus on a thermal printing head a yellow coloured image, having good light fastness, was formed rapidly.

**Claims for the Contracting States: BE CH DE FR GB IT LI LU NL SE**

1. A compound of the general formula (I):

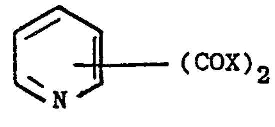

I

wherein X is:

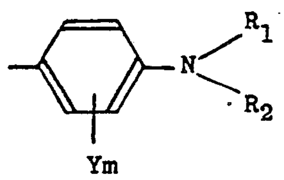, 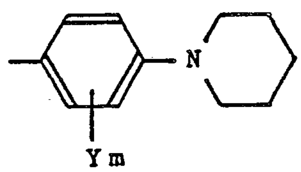, 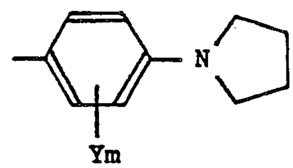,

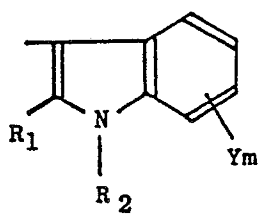, 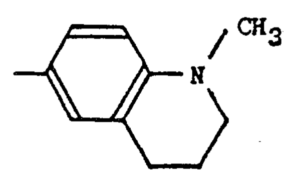, 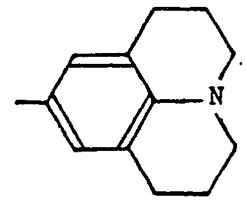,

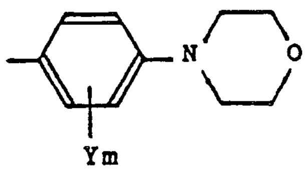, 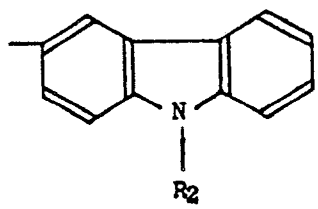 or 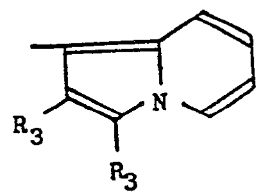;

where Y is $C_1$ to $C_6$ alkyl, substituted $C_1$ to $C_6$ alkyl, $C_1$ to $C_6$ alkoxy, substituted $C_1$ to $C_6$ alkoxy, $C_1$ to $C_6$ alkyl dialkylamino, phenyl, substituted phenyl, benzyl, substituted benzyl, halogen, a fused benzene ring or a substituted fused benzene ring;

m is O or an integer from 1 to 4; and

each $R_1$, $R_2$ and $R_3$ is independently $C_1$ to $C_{10}$ alkyl, substituted $C_1$ to $C_{10}$ alkyl, phenyl, substituted phenyl, benzyl, substituted benzyl or $C_5$ or $C_6$ cycloalkyl; and

wherein the substituent radical(s) in the "substituted" alkyl, alkoxy, phenyl, fused benzene ring or benzyl radical(s) is (are) $C_1$ to $C_{10}$ alkyl, halogen substituted $C_1$ to $C_4$ alkyl, $C_1$ to $C_4$ alkoxy, $C_1$ to $C_4$ alkyl dialkylamino, halogen or cyano.

2. A compound a claimed in Claim 1 wherein X is

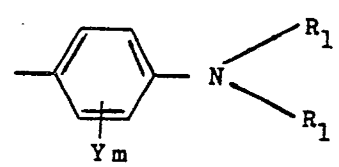

wherein each $R_1$ is a $C_1$ to $C_4$ alkyl group and Y and m are as defined in claim 1.

3. A compound as claimed in claim 2 wherein Y is chlorine.

4. A Compound as claimed in either claim 2 or claim 3 wherein m is 1.

5. 2,6-Bis(N,N-dimethyl-4'-aminobenzoyl)pyridine.

6. 2,6-Bis(N,N-di-*n*-butyl-4'-amino-2'-chlorobenzoyl)pyridine.

7. A method for making a compound as claimed in any one of claims 1 to 6 which comprises reacting a pyridine dicarboxylic acid chloride or bromide with a compound of the formula XH where X is as defined in any one of claims 1 to 4, in an organic solvent in the presence of a Friedel-Crafts catalyst.

8. A method of making an ortho compound as claimed in any one of claims 1 to 4 which comprises treating a leuco compound of the formulas (IV) to (VII):

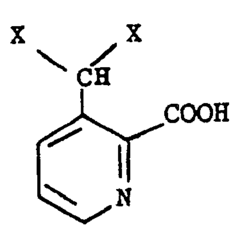

IV

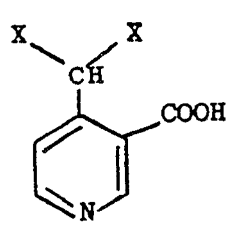

V

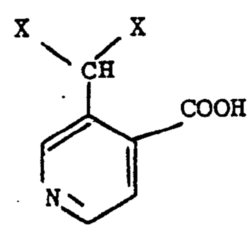

VI

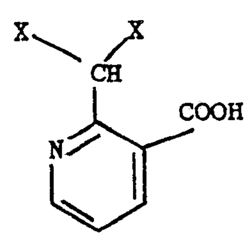

VII

wherein X is:

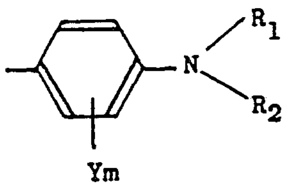 , 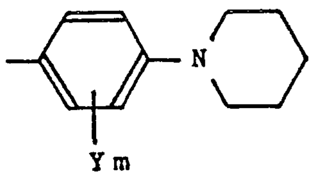 , 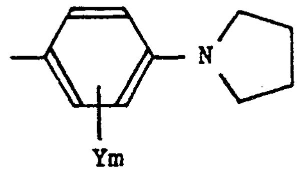 ,

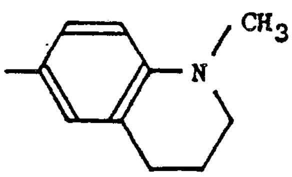 , 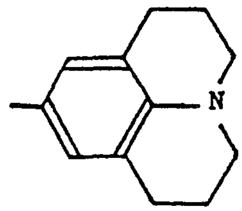 ,

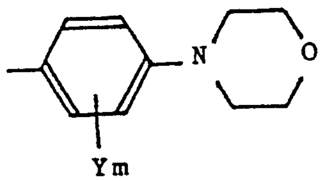 or 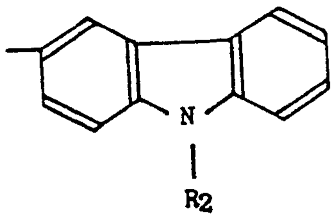

where Y, m, $R_1$, $R_2$ and $R_3$ are as defined in any one of claims 1 to 4, with an acid anhydride, and subsequently with an oxidising agent.

9. A pressure-sensitive or heat-sensitive recording material comprising at least one compound as claimed in any one of claims 1 to 6 as a colour former.

10. Manifold pressure-sensitive recording material which comprises an upper sheet carrying on the lower surface thereof a coating of a microencapsulated solution of a chromogenic composition, a lower sheet carrying on the upper surface thereof a coating comprising an electron-accepting co-reactant and, optionally, one or more intermediate sheets each of which has a coating on its upper surface an electron accepting co-reactant and on its lower surface a microencapsulated solution of a chromogenic composition, characterized in that a compound as claimed in any one of claims 1 to 6 is included as a colour former in the chromogenic composition(s).

11. Thermally responsive record material comprising a sheet substrate having at least one surface thereof a coating comprising a finely divided solid chromogenic composition, a finely divided solid co-reactant and, optionally, a finely divided solid thermofusible wax, characterized in that the chromogenic composition includes at least one compound as claimed in any one of claims 1 to 6.

12. Record material as claimed in either claim 10 or claim 11 characterized in that the chromogenic composition additionally includes at least one colour former other than as claimed in claim 1 such that, on reaction with the co-reactant, a perceived black image is produced.

**Claims for the Contracting State: AT**

1. A pressure-sensitive or heat-sensitive recording material comprising as a colour former at least one compound of the general formula (I):

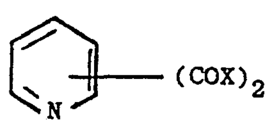 I

wherein X is:

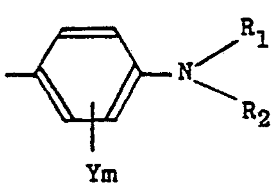 , 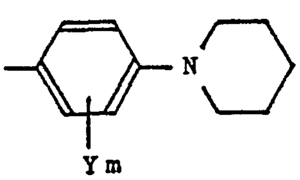 , 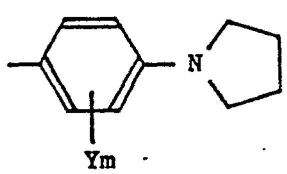 ,

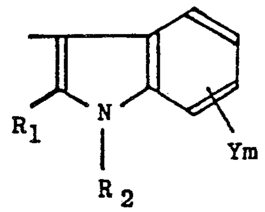,
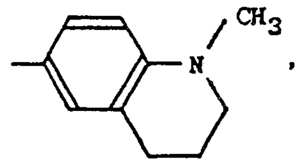,
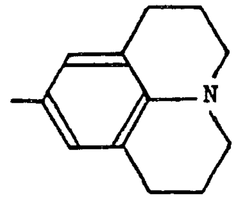,

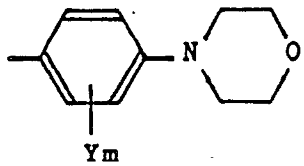,
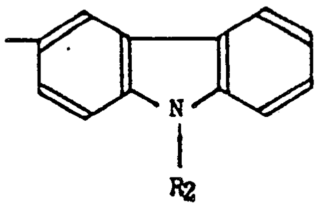 or
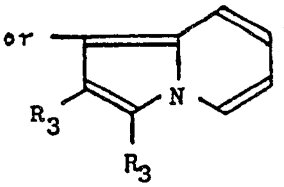;

where Y is $C_1$ to $C_6$ alkyl, substituted $C_1$ to $C_6$ alkyl, $C_1$ to $C_6$ alkoxy, substituted $C_1$ to $C_6$ alkoxy, $C_1$ to $C_6$ alkyl dialkylamino, phenyl, substituted phenyl benzyl, substituted benzyl, halogen, a fused benzene ring or a substituted fused benzene ring;

m is O or an integer from 1 to 4; and

Each $R_1$, $R_2$ and $R_3$ is independently $C_1$ to $C_{10}$ alkyl, substituted $C_1$ to $C_{10}$ alkyl, phenyl, substituted phenyl, benzyl, substituted benzyl or $C_5$ or $C_6$ cycloalkyl; and

wherein the substituent radical(s) in the "substituted" alkyl, alkoxy, phenyl, fused benzene ring or benzyl radical(s) is (are) $C_1$ to $C_{10}$ alkyl, halogen substituted $C_1$ to $C_4$ alkyl, $C_1$ to $C_4$ alkyl dialkylamino, halogen or cyano.

2. Record material as claimed in Claim 1 wherein X is

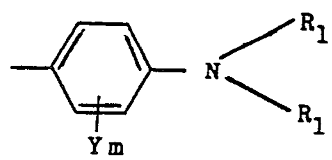

wherein each $R_1$ is a $C_1$ to $C_4$ alkyl group and Y and m are as defined in claim 1.

3. Record material as claimed in claim 2 wherein Y is chlorine.

4. Record material as claimed in either claim 2 or claim 3 wherein m is 1.

5. Record material as claimed in claim 1 wherein the compound(s) of the general formula (I) is or includes 2,6-Bis(N,N-dimethyl-4′-aminobenzoyl)pyridine.

6. Record material as claimed in claim 1 wherein the compound(s) of the general formula (I) is or includes 2,6-Bis(N,N-di-*n*-butyl-4′-amino-2′-chlorobenzoyl)pyridine.

7. A method of making a compound of the general formula (I) as defined in claim 1 which comprises reacting a pyridine dicarboxylic acid chloride or bromide with a compound of the formula XH where X is as defined in any one of the claims 1 to 4, in an organic solvent in the presence of a Friedel-Crafts catalyst.

8. A method of making an ortho compound of the general formula (I) as defined in any one of claims 1 to 4 which comprises treating a leuco compound of the formula (IV) to (VII):

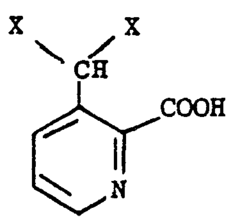
IV

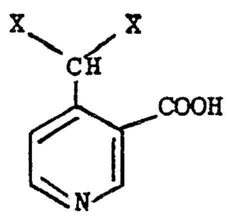
V

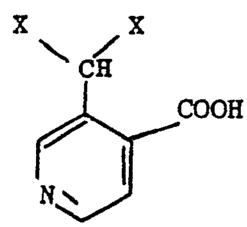
VI

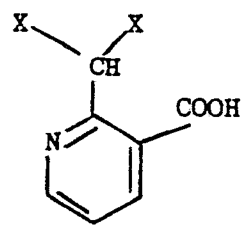
VII

wherein X is

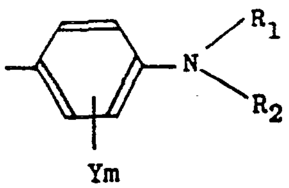
,
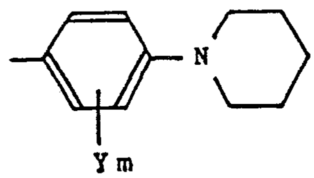
,
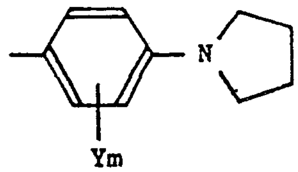
,

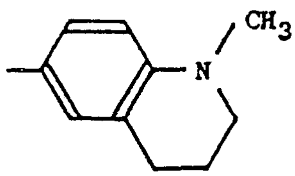
,
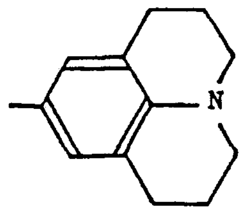
,

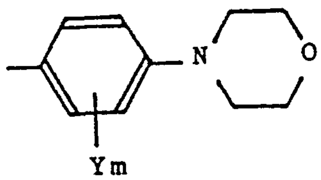
or
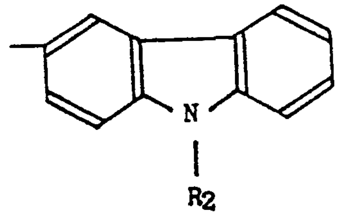

where Y, m, $R_1$, $R_2$ and $R_3$ are as defined in any of claims 1 to 4, with an acid anhydride, and subsequently with an oxidising agent.

9. Manifold pressure-sensitive recording material as claimed in claim 1 which comprises an upper sheet carrying on the lower surface thereof a coating of a microencapsulated solution of a chromogenic composition, a lower sheet carrying on the upper surface thereof a coating comprising an electron-accepting co-reactant and, optionally, one or more intermediate sheets each of which has a coating on its upper surface an electron accepting co-reactant and on its lower surface a microencapsulated solution of a chromogenic composition, characterized in that a compound of the general formula (I) as defined in any one of claims 1 to 6 is included as a colour former in the chromogenic composition(s).

10. Thermally responsive record material as claimed in claim 1 comprising a sheet substrate having on at least one surface thereof a coating comprising a finely divided solid chromogenic composition, a finely divided solid co-reactant and, optionally, a finely divided solid thermofusible wax, characterized in that the chromogenic composition includes at least one compound of the general formula (I) as defined in any one of claims 1 to 6.

11. Record material as claimed in either claim 9 or claim 10 characterized in that the chromogenic composition additionally includes at least one colour former other than of the general formula (I) as defined in claim 1 such that, on reaction with the co-reactant, a perceived black image is produced.

**Patentansprüche für die Vertragsstaaten: BE CH DE FR GB IT LI LU NL SE**

1. Eine Verbindung der allgemeinen Formel (I)

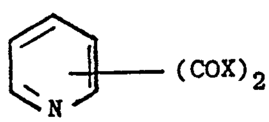
I

mit X =

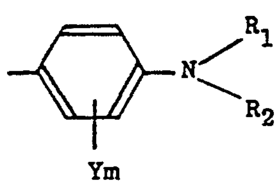
,
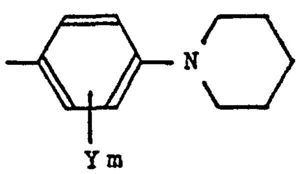
,
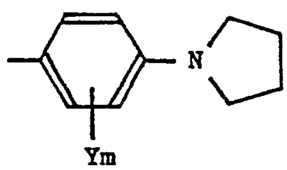
,

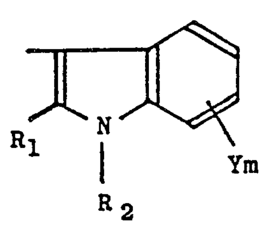
,
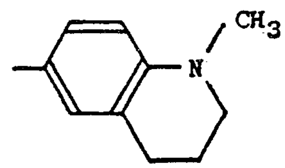
,
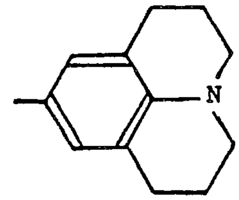
,

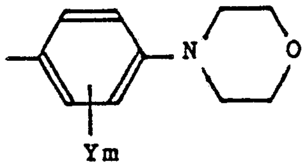
,
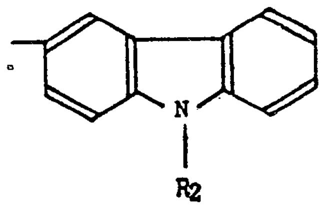
oder
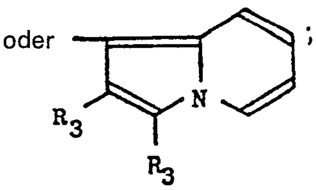
;

wobei Y eine $C_1$ bis $C_6$ Alkylgruppe, eine substituierte $C_1$ bis $C_6$ Alkylgruppe, eine $C_1$ bis $C_6$ Alkoxygruppe, eine substituierte $C_1$ bis $C_6$Alkoxygruppe, eine $C_1$ bis $C_6$ Alkyldialkylaminogruppe, einen Phenylrest, einen substituierten Phenylrest, einen Benzylrest, einen substituierten Benzylrest, ein Halogenatom oder ein kondensierter Benzolring oder ein substituierter kondensierter Benzolring ist; m = 0 oder ganze Zahl von 1 bis 4 ist; und

jedes $R_1$, $R_2$ und $R_3$ unabhängig voneinander eine $C_1$ bis $C_6$ Alkylgruppe, eine substituierte $C_1$ bis $C_{10}$ Alkylgruppe, ein Phenylrest, ein substituierter Phenylrest, ein Benzylrest, ein substituierter Benzylrest oder ein $C_5$ oder $C_6$ Cycloalkylrest ist und

worin die Substitutionsreste in den "substituierten" Alkyl-, Alkoxy-, Phenyl-, kondensierte Benzolring oder Benzylrest(e) eine $C_1$ bis $C_{10}$ Alkyl-, eine halogensubstituierte $C_1$ bis $C_4$ Alkyl-, eine $C_1$ bis $C_4$ Alkoxy-, $C_1$ bis $C_4$ Alkyldialkylamino-, Halogen-oder Cyangruppe darstellt bzw. darstellen.

2. Eine Verbindung nach Anspruch 1, worin gleich

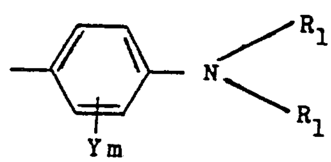

ist und jedes $R_1$ eine $C_1$ bis $C_4$ Alkylgruppe bedeutet und Y und m die in Anspruch 1 definierte Bedeutung aufweisen.

3. Verbindung nach Anspruch 2, in der Y gleich Chlor ist.

4. Eine Verbindung nach Anspruch 2 oder 3, in der m gleich 1 ist.

5. 2,6-Bis(N,N-dimethyl-4'-aminobenzoyl)pyridin.

6. 2,6-Bis(N,N-di-n-butyl-4'-amino-2'-chlorobenzoyl)pyridin.

7. Ein Verfahren zur Herstellung der Verbindungen nach Anspruch 1 bis 6, umfassend die Umsetzung eines Pyridindicarboxylsäurechlorids oder Bromids mit einer Verbindung der Formel XH, in der X die in den Ansprüchen 1 bis 4 definierte Bedeutung hat, in einem organischen Lösungsmittel im Beisein eines Friedel-Crafts-Katalysators.

8. Ein Verfahren zur Herstellung einer Orthoverbindung nach einem der Ansprüche 1 bis 4, umfassend das Behandeln einer Leukoverbindung der Formeln (IV) bis (VIII)

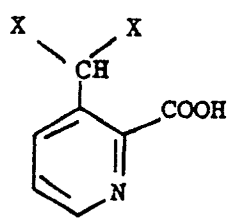
IV

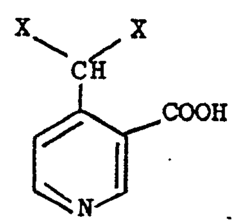
V

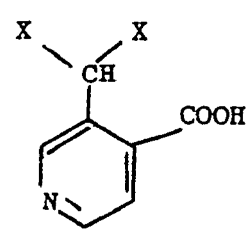
VI

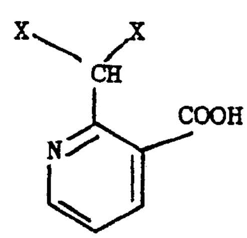
VII

mit X =

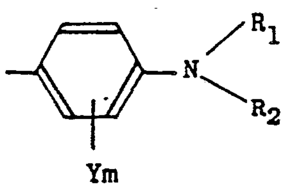 , 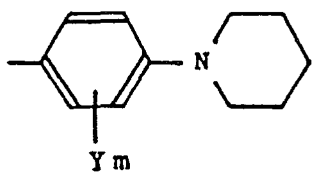 , 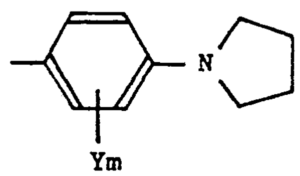 ,

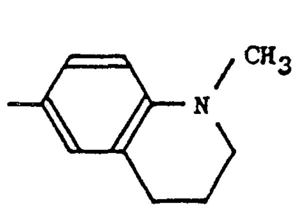 , 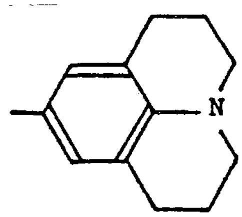 ,

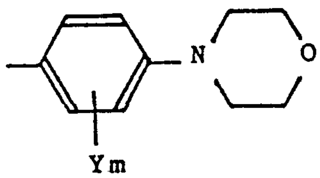 oder 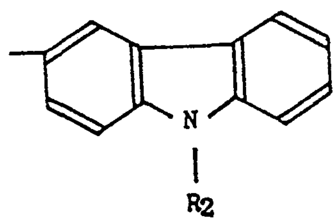

wobei Y, m, $R_1$, $R_2$ und $R_3$ die in den Ansprüchen 1 bis 4 definierten Bedeutungen haben mit einem Säureanhybrid und dem darauffolgenden mit einem oxidierenden Agens.

9. Ein druckempfindliches oder wärmeempfindliches Aufzeichnungsmaterial, das zumindest eine Verbindung der Ansprüche 1 bis 6 als Farbbildner enthält.

10. Ein mehrschichtiges druckempfindliches Aufzeichnungsmaterial, umfassend ein oberes Blatt, das auf seiner unteren Oberfläche mit einer mokroverkapselten Lösung einer chromogenen Zusammensetzung überzogen ist, einem unteren Blatt, das auf seiner oberen Oberfläche mit einem Überzug beschichtet ist, der einen elektronenaufnehmenden Koreaktanten enthält und gegebenenfalls ein oder meherer Zwischenblätter, die an ihrer oberen Seite mit einem elektronenaufnehmenden Koreaktanten und auf ihrer unteren Seite mit einer mikroverskapselten Lösung eines chromogenen Materials beschichtet sind, dadurch gekennzeichnet, daß die chromogene Zusammensetzung(en) eine Verbindung der Ansprüche 1 bis 6 als Farbbildner enthalten.

11. Warmeempfindliches Aufzeichnungsmaterial, umfassend ein Blattsubstrat, das zumindest auf einer Oberfläche mit einem Überzug beschichtet ist, der eine feinverteilten chromogene Zusammensetzung, einen feinverteilten, festen Koreaktanten und gegebenefalls ein feinverteilten, festen thermoverschmelzbares Wachs enthält, dadurch gekennzeichnet, daß die chromogene Zusammensetzung mindestens eine Verbindung der Ansprüche 1 bis 6 enthält.

12. Aufzeichnungsmaterial nach Anspruch 10 oder 11, dadurch gekennzeichnet, daß die chromogene Zusammensetzung zusätzlich mindestens einen Farbbildner enthält, der nicht von Anspruch 1 beansprucht wird, so daß Reaktion mit dem Koreaktanten ein schwarz erscheinendes Bild bildet.

**Patentansprüche für den Vertragsstaat: AT**

1. Ein druckempfindliches oder hitzeempfindliches Aufzeichnungsmaterial, welches als Farbbildner zumindest eine der Verbindungen der allgemeinen Formel (I) enthält

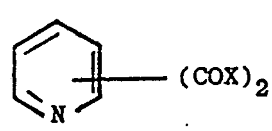 I

mit X =

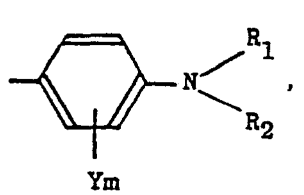 , 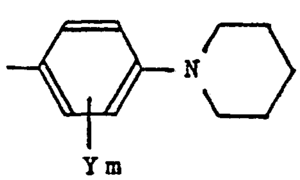 , 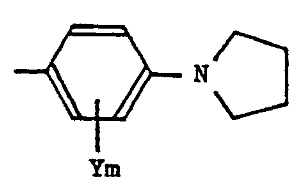 ,

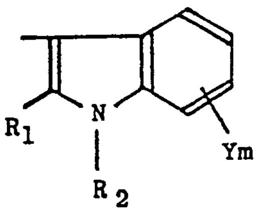, 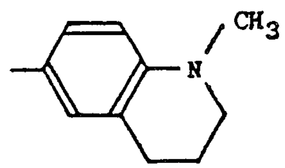, 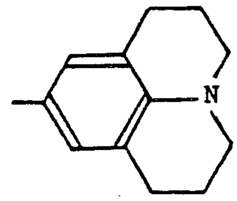,

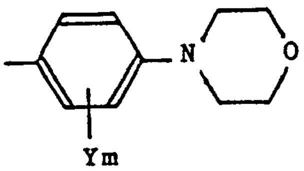, 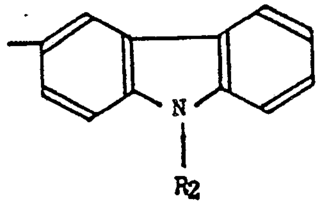 oder 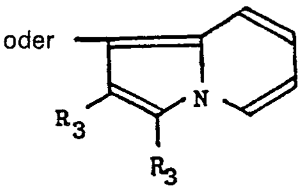;

wobei Y eine $C_1$ bis $C_6$ Alkyl-, substituierte $C_1$ bis $C_6$ Alkyl-, $C_1$ bis $C_6$ Alkoxy-, substitierte $C_1$ bis $C_6$ Alkoxy-, $C_1$ bis $C_6$ Alkyldialkylamino-, Phenyl, substituierte Phenyl-, Benzyl-, substituierte Benzylgruppe, Halogenatom, ein kondensierter Benzolring oder ein substituierter kondensierter Benzolring bedeutet, m gleich O oder eine ganze Zahl von 1 bis 4 ist und jedes $R_1$, $R_2$ und $R_3$ unabhängig voneinander eine $C_1$ bis $C_{10}$ Alkyl-, Substituierte $C_1$ bis $C_{10}$ Alkyl-, Phenyl-, substituierte Phenyl-, Benzyl-, substituierte Benzyl-, oder $C_5$ oder $C_6$ Cycloalkylgruppe ist und worin die Substituentengruppen in den "substituierten" Alkyl-, Alkoxy-, Phenylgruppen, kondensierten Benzolringen oder Benzylreste eine $C_1$ bis $C_{10}$ Alkyl-, eine halogen-substituierte $C_1$ bis $C_4$ Alkyl-, $C_1$ bis $C_4$ Alkoxy-, $C_1$ bis $C_4$ Alkyldialkylaminogruppe, Halogenatom oder Cyanogruppe bedeutet.

2. Aufzeichnungsmaterial nach Anspruch 1, mit X =

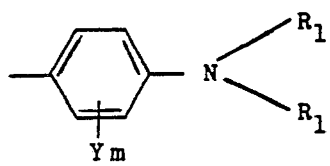

wobei jedes $R_1$ eine $C_1$ bis $C_5$ Alkylgruppe ist und Y und m die in Anspruch 1 definierte Bedeutung haben.

3. Aufzeichnungsmaterial nach Anspruch 2, worin Y gleich Chlor ist.

4. Aufzeichnungsmaterial nach Anspruch 2 oder 3, worin m gleich 1 izt.

5. Aufzeichnungsmaterial nach Anspruch 1, worin die Verbindung(en) der allgemeinen Formel (I) 2,6-Bis(N,N-dimethyl-4'-aminobenzoyl)pyridin ist oder enthält.

6. Aufzeichnungsmaterial nach Anspruch 1, worin die Verbindung(en) der allgemeinen Formel (I) 2,6-Bis(N,N-di-n-butyl-4'amino-2'-chlorbenzoyl)pyridin ist oder enthält.

7. Ein Verfahren zur Herstellung einer der allgemeinen Formel (I) wie in Anspruch 1 definiert, umfassend die Umsetzung eines Pyridindicarboxylsäurechlorides oder Bromides mit einer Verbindung der Formel HX, wobei X die in einem der Ansprüche 1 bis 4 definierte Bedeutung hat, in einem organischen Lösungsmittel im Beisein eines Friedel-Crafts-Katalysators.

8. Ein Verfahren zur Herstellung einer Orthoverbindung der allgemeinen Formel (I), wie sie in einem der Ansprüche 1 bis 4 definiert ist, umfassend die Behandlung einer Leukoverbindung der Formeln (IV) bis (VII)

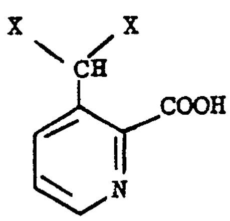

IV

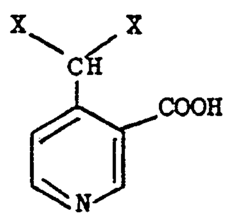

V

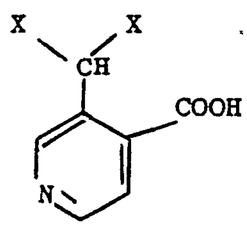

VI

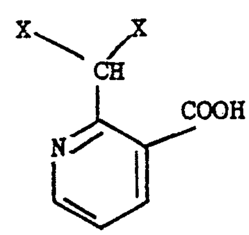

VII

mit X gleich

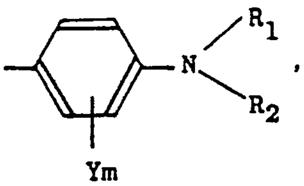, 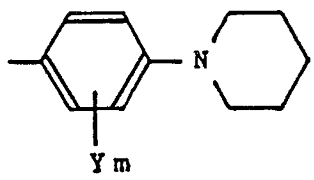, 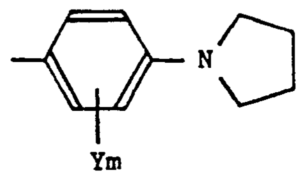,

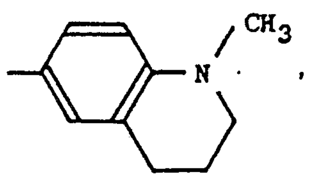, 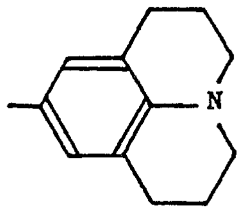,

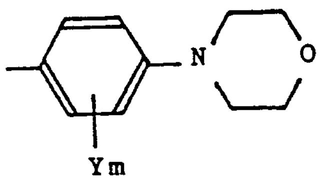 oder 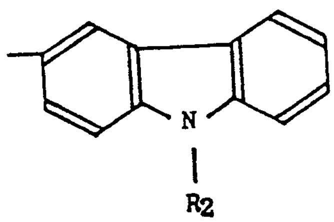

wobei Y, m, $R_1$, $R_2$ und $R_3$ die in den Ansprüchen 1 bis 4 definierte Bedeutung haben, mit einem Säureanhydrid und anschließender Behandlung mit einem oxidierenden Agens.

9. Mehrschichtiges, druckempfindliches Aufzeichnungsmaterial nach Anspruch 1, umfassend ein oberes Blatt, das auf seiner unteren Oberfläche mit einer mikroverkapselten Lösung einer chromogenen Zusammensetzung beschichtet ist, einem unteren Blatt, dessen obere Oberfläche mit einer Beschichtung überzogen ist, die einen obere Oberfläche mit einer Beschichtung überzogen ist, die einen elektronenaufnehmenden Koreaktanten enthält und gegebenenfalls ein oder mehrere Zwischenblätter, die auf ihrer oberen Seite einen elektronaufnehmenden Koreaktanten und auf ihrer unteren Seite mit einer mikroverkapselten Lösung einer chromogenen Zusammensetzung beschichtet sind, dadurch gekennzeichnet, daß die chromogene(n) Zusammensetzung(en) als Farbbildner eine Verbindung der allgemeinen Formel (I) enthalten, wie sie in den Ansprüchen 1 bis 6 definiert ist.

10. Wärmeempfindliches Aufzeichnungsmaterial nach Anspruch 1, umfassend ein Blattsubstrat, das mindestens auf einer Oberfläche eine Beschichtung hat, die eine feinverteilte, feste chromogene Zusammensetzung, einen feinverteilten, festen Koreaktanten und gegebenenfalls ein feinverteiltes, wärmeverschmelzbares Wachs enthält, dadurch gekennzeichnet, daß die Zusammensetzung zumindest eine Verbindung der in den Ansprüchen 1 bis 6 definierten allgemeinen Formel (I) enthält.

11. Aufzeichnungsmaterial nach einem der Ansprüche 9 oder 10, dadurch gekennzeichnet, daß die chromogene Zusammensetzung zumindest einen Farbbildner enthält, der nicht die in Anspruch 1 definierte allgemeine Formel (I) hat, damit bei der Reaktion in dem Koreaktanten ein schwarz erscheindendes Bild entsteht.

**Revendications pour les Etats contractants: BE CH DE FR GB IT LI LU NL SE**

1. A composé de formule généralé (I):

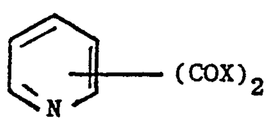 I

dans laquelle X représente:

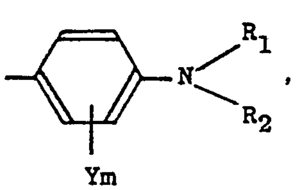, 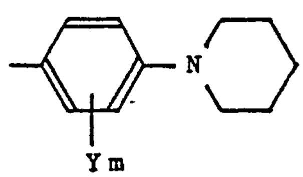, 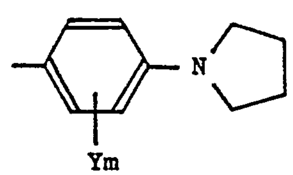,

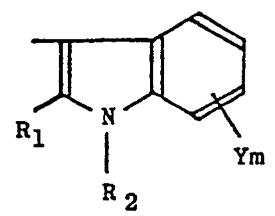 , 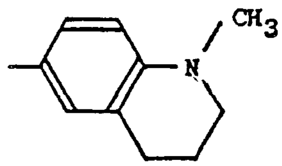 , 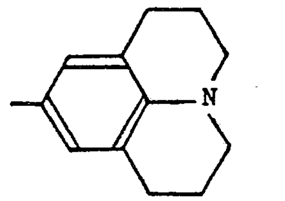 ,

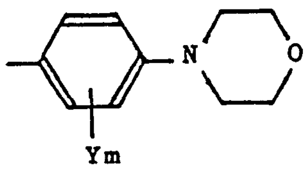 , 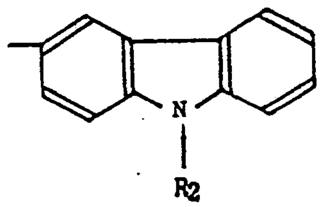 ou 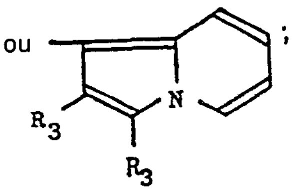 ;

dans lesquelles Y représente un groupe alkyle en $C_1$ à $C_6$, un groupe alkyle $C_1$—$C_6$ substitué, un groupe alkoxy en $C_1$ à $C_6$, un groupe alkyle $C_1$—$C_6$, un groupe alkyle dialkylamino en $C_1$ à $C_6$, un group phényle, phényle substitué, benzyle, benzyle substitué, halogène, un cycle benzénique accolé ou un cycle benzénique substitué accolé; m est égal à 0 ou à un nombre entier de 1 à 4; et $R_1$, $R_2$ et $R_3$ représentant chacun, de façon indépendante, un groupe alkyle en $C_1$ à $C_{10}$, un groupe alkyle $C_1$—$C_{10}$ substitué, un groupe phényle, phényle substitué, benzyle, benzyle substitué, ou cycloalkyle en $C_5$ ou $C_6$; le radical (ou les radicaux) substituant dans les groupes alkyle, alkoxy, phényle, cycle benzénique accolé ou benzyle "substitués" étant un groupe (ou des groupes) alkyle en $C_1$ à $C_{10}$, alkyle en $C_1$ à $C_4$ substitué par hologène, alkoxy en $C_1$ à $C_4$. alkyle dialkylamino en $C_1$ à $C_4$, halogène ou cyano.

2. Composé selon la revendication 1, caractérisé en ce que X répond à la formule

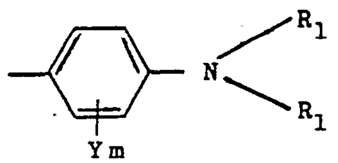

dans laquelle chaque $R_1$ est un groupe alkyle en $C_1$ à $C_4$ et Y et m sont définis comme dans la revendication 1.

3. Composé selon la revendication 2, caractérisé en ce que Y représente le chlore.

4. Composé selon la revendication 2 ou 3, caractérisé en ce que m est égal à 1.

5. 2,6-Bis(N,N-diméthyl-4'-aminobenzoyl)pyridine.

6. 2,6-Bis(N,N-di-*n*-butyl-4'-amino-2'-chlorobenzoyl)pyridine.

7. Procédé de préparation d'un composé selon l'une quelconque des revendications 1 à 6, comprenant l'étape consistant à faire réagir un chlorure ou bromure d'acide dicarboxylique de pyridine avec un composé de formule XH où X est tel que défini dans l'une quelconque des revendications 1 à 4, dans un solvant organique, en présence d'un catalyseur Friedel-Crafts.

8. Procédé de préparation d'un composé ortho selon l'une quelconque des revendications 1 à 4, comprenant les étapes consistant à traiter un composé leuco répondant aux formules (IV) à (VII)

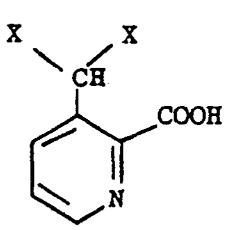
IV

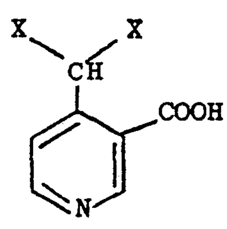
V

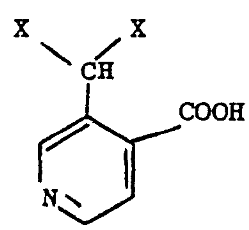
VI

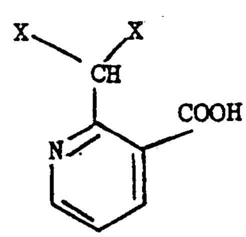
VII

dans lesquelle X répresente:

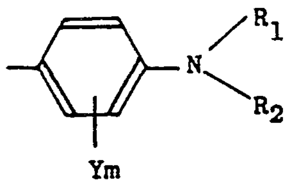, 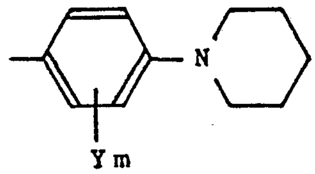, 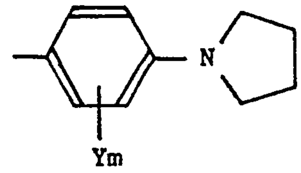,

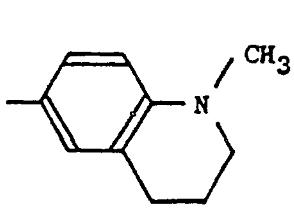, 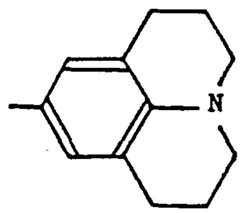,

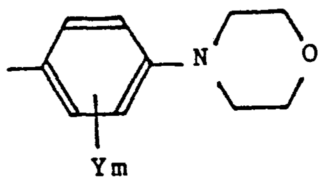 ou 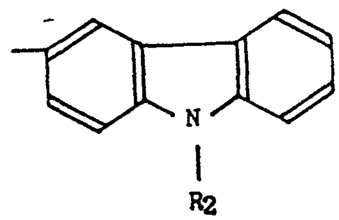

où Y, m, $R_1$, $R_2$, $R_3$ sont tels que définis dans l'une quelconque des revendications 1 à 4, avec un anhydride d'acide, et ensuite avec un agent oxydant.

9. Matériau d'enregistrement sensible à la pression ou à la chaleur, comprenant au moins un composé selon l'une quelconque des revendications 1 à 6, comme formateur de couleur.

10. Matériau d'enregistrement pour copies multiples, sensible à la pression, comprenant une feuille supérieure portant, sur sa face inférieure, un revêtement formépar la solution, renfermée dans des microcapsules, d'une composition chromogénique, une feuille inférieure portant sur sa face supérieure un revêtement comprenant un co-réactant accepteur d'électrons et, éventuellement, une ou plusieurs feuilles intermédiaires dont chacune est munie d'un revêtement constitué sur sa face supérieure, par un co-réactant accepteur d'électrons et sur sa face intérieure, par une solution, renfermée dans des microcapsules, d'une composition chromogénique, matériau caractérisé en ce qu'un composé présent selon l'une quelconque des revendications 1 à 6 est present, comme formateur de couleur, dans revendications 1 à 6 est présent, comme formateur de couleur, dans la ou les compositions chromogéniques.

11. Matériau d'enregistrement thermo-sensible, comprenant un substrat sous forme de feuille portant, sur au moins l'une de ses faces, un revêtement comprenant une composition chromogénique solide, finement divisée, un co-réactant solide, finement divisée, matériau caractérisé en ce que la composition chromogénique comprend au moins un composé selon l'une quelconque des revendications 1 à 6.

12. Matériau d'enregistrement selon la revendication 10 ou 11, caractérisé en ce que la composition chromogénique comprend en outre au moins un formateur de couleur autre que celui revendiqué dans la revendication 1, tel que, par réaction avec le co-réactant, il soit produit une image noire perçue.

**Revendications pour l'Etat contractant: AT**

1. Matériau d'enregistrement sensible à pression ou à la chaleur, comprenant, comme formateur de couleur, au moins un composé de formule générale (I):

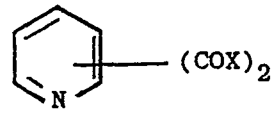 I

dans laquelle X représente:

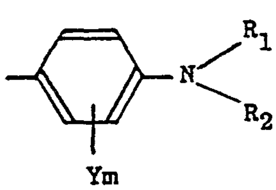, 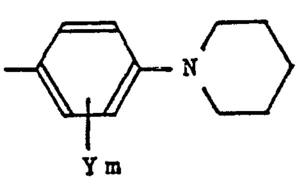, 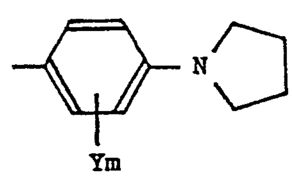,

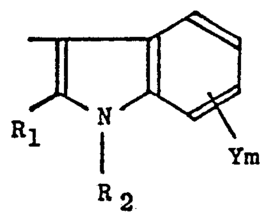 , 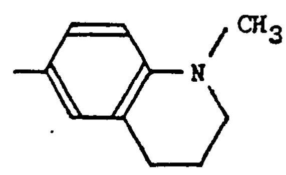 , 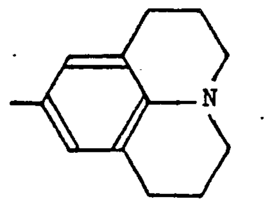 ,

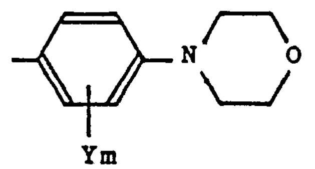 , 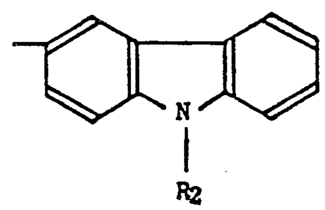 ou 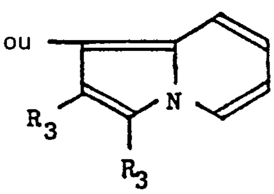 ;

dans lesquelles Y représente un groupe alkyle en $C_1$ à $C_6$, un groupe alkyle substitué, en $C_1$ à $C_6$, un groupe alkoxy en $C_1$ à $C_6$, un groupe alkoxy en $C_1$ à $C_6$, un groupe alkoxy substitué, en $C_1$ à $C_6$, un groupe alkyl dialkylamino en $C_1$ à $C_6$, un groupe phényle, phényle substitué, benzyle, benzyle substitué, halogène un cycle benzénique accollé ou un cycle benzénique substitué accollé; m est égal à 0 ou à un nombre entier de 1 à 4; et $R_1$, $R_2$ et $R_3$ représentent chacun, de façon, indépendante, un groupe alkyle en $C_1$ à $C_{10}$, un groupe alkyle substitué, en $C_1$ à $C_{10}$, un groupe phényle, phényle substitué, benzyle, benzyle substitué, ou cycloalkyle en $C_5$ ou $C_6$; et où le radical (ou les radicaux) substituants dans les groupes alkyle, alkoxy, phényle, cycle benzénique accollé ou benzyle "substitués" sont un groupe alkyle en $C_1$ à $C_{10}$, un groupe alkyle en $C_1$ à $C_4$ substitué par halogéne, un groupe alkoxy en $C_1$ à $C_4$, un groupe alkyle dialkylamino en $C_1$ à $C_4$, un groupe halogène ou cyano.

2. Composé selon la revendication 1, caractérisé en ce que X répond à la formule

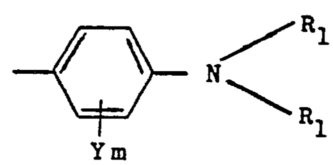

dans laquelle chaque $R_1$ est un groupe alkyle en $C_1$ à $C_5$ et Y et m sont définis comme dans la revendication 1.

3. Matériau d'enregistrement selon de revendication 2, caractérisé en ce que Y est du chlore.

4. Matériau d'enregistrement selon des revendications 2 ou 3, caractérisé en ce que m est égal à 1.

5. Matériau d'enregistrement selon de revendication 1, caractérisé en ce que le ou les composés de formule générale (I) est constitué par ou comprennent la 2,6-Bis(N,N-diméthyl-4'-aminobenzoyl)pyridine.

6. Matériau d'enregistrement selon la revendication 1, caractérisé en ce que le ou les composés de formulé générale (I) sont constitués ou comprennent la 2,6-Bis(N,N-di-*n*-butyl-4'-amino-2'-chlorobenzoyl)pyridine.

7. Procédé de préparation d'un composé de formule générale (I) selon la revendication 1, comprenant la réaction d'un chlorure ou bromure d'acide dicarboxylique de pyridine avec un composé de formule XH où X est défini comme dans l'une quelconque des revendications 1 à 4, dans un solvant organique, en présence d'un catalyseur Friedel-Crafts.

8. Procédé de préparation d'un composé ortho de formule générale (I), comme défini dans l'une quelconque des revendications 1 à 4, comprenant le traitement d'un composé leuco des formules (IV) à (VII)

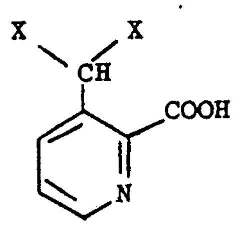
IV

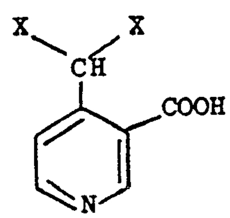
V

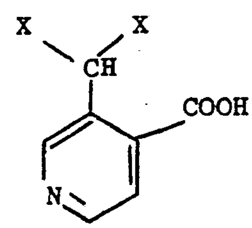
VI

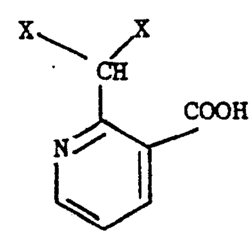
VII

dans lesquelle X représente:

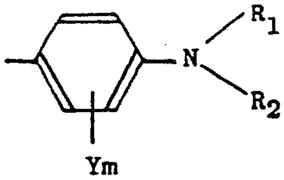, 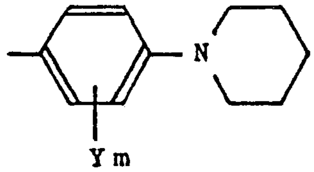, 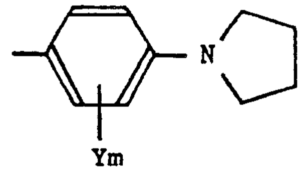,

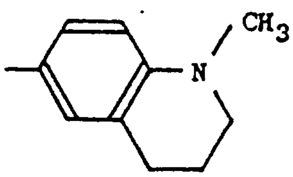, 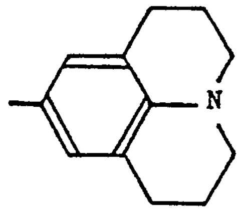,

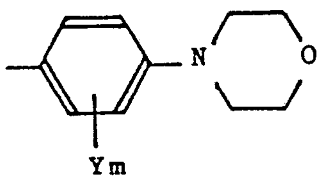 ou 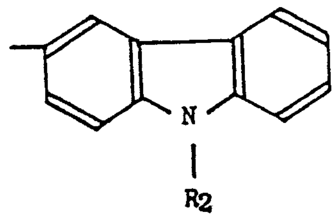

où Y, m, $R_1$, $R_2$ et $R_3$ sont définis comme dans l'une quelconque des revendications 1 à 4, avec un anhydride d'acide et ensuite, avec un agent oxydant.

9. Matériau d'enregistrement sensible à la pression, pour copie, selon la revendication 1, constitué par une feuille supérieure portant, sur sa face inférieure, un revêtement formé par solution, renfermée dans des microcapsules, d'une composition chromogénique, une feuille inférieure portant sur sa face supérieure un revêtement comprenant un co-réactant accepteur d'électrons et, éventuellement, une ou plusieurs feuilles intermédiaires dont chacune est munie d'un revêtement constitué sur sa face supérieure, par un co-réactant accepteur d'électrons et sur sa face inférieure, par une solution, renfermée dans des microcapsules, d'une composition chromogénique, matériau caractérisé en ce qu'un composé de formule générale (I) selon l'une quelconque des revendications 1 à 6 est compris, comme formateur de couleur, dans la ou les compositions chromogéniques.

10. Matériau d'enregistrement thermosensible selon la revendication 1, comprenant un substrat de feuille portant, sur au moins l'une de ses faces, un revêtement, comprenant une composition chromogenique solide, finement divisés, un co-réactant solide, finement divisé et, éventuellement, une cire thermofusible solide, finement divisée, matériau caractérisé en ce que la composition chromogénique comprend au moins un composé de formule générale (I) selon l'une quelconque des revendications 1 à 6.

11. Matériau d'enregistrement selon les revendications 9 ou 10, caractérisé en ce que la composition chromogénique comprend en outre au moins un formateur de couleur autre que celui répondant à la formule générale (I), comme défini dans la revendication 1, tel que, par réaction avec le co-réactant, il soit produit une image noire perçue.